# EUROPEAN PATENT APPLICATION

(11) **EP 2 471 473 A1**
(43) Date of publication of application: **04.07.2012**
(21) Application number: 10382362.1
(22) Date of filing: 29.12.2010
(51) Int. Cl.: A61B 17/29

(54) **Apparatus for laparoscopic surgery**

(71) Applicant: Consorcio para la Gestion del Centro de Cirugia de Minima Invasion, 10071 Caceres (ES); Instituto de Biomecanica de Valencia, 46022 Valencia (ES); Universidad De Extremadura, 06071 Badajoz (ES)
(72) Inventor: Sánchez Margallo, Francisco Miguel, E-10071 Cáceres (ES); Pagador Carrasco, José Blas, E-10071 Cáceres (ES); Lucas Hernández, Marcos, E-10071 Cáceres (ES); Sánchez Margallo, Juan Alberto, E-10071 Cáceres (ES); Pérez Duarte, Francisco Julián, E-10071 Cáceres (ES); Díaz-Güemes Martín-Portugués, Idoia, E-10071 Cáceres (ES); Usón Gargallo, Jesús, E-10071 Cáceres (ES); Oltra Pastor, Alfonso, E- 46022 Valencia (ES); Castelló Mercé, Purificación, E- 46022 Valencia (ES); Fayos Sancho, Juan, E- 46022 Valencia (ES); Atienza Vicente, Carlos Manuel, E- 46022 Valencia (ES); Bermejo Bosch, Ignacio, E- 46022 Valencia (ES); García Moruno, Lorenzo, E-06470 Guareña - Badajoz (ES); Rodríguez Salgado, David, E-06011 Badajoz (ES); Gil Peña, Francisco José, E- 06200 Almendralejo - Badajoz (ES); Benito Rodríguez, Ángel, E-06001 Badajoz (ES)
(74) Representative: ABG Patentes, S.L.

(57) **Abstract**

The present invention relates to an opening and closing surgical apparatus for laparoscopic surgery with ergonomic technical features which provide important improvements in the activity of surgeons performing this surgical technique minimally invasive. This ergonomic capacity is due to a particular form of actuating the opening and closing end tool.

## Description

### Object of the Invention

The present invention relates to an opening and closing surgical apparatus for laparoscopic surgery with ergonomic technical features which provide important improvements in the activity of surgeons performing this surgical technique minimally invasive. This ergonomic capacity is due to a particular form of actuating the opening and closing end tool.

### Background of the Invention

Minimally invasive surgery (MIS) techniques have been rapidly introduced into common surgical practice and it is increasing more frequent to find indications for which MIS has replaced traditional surgery techniques due to the advantages it has for the patient both during the surgery and during postoperative recovery.

In recent years, research relating to interventions of this type has been based on improving the technique and evaluating the cost-effectiveness relationship. However, the problems experienced by surgeons due to muscle fatigue and even paresthesia related to the ergonomic deficiency of the instruments used are known.

In works related to the ergonomics of laparoscopic surgery, one of the first issues studied was the difference between conventional and laparoscopic surgery in terms of the postural attitude and the onset of physical and mental fatigue. Smith et al. and Berguer *et al.* concluded that laparoscopy involves greater physical stress and also requires a longer execution time. Other works were focused on the study of the movements of the upper extremity and the pain that the surgeon suffers during laparoscopic and conventional procedures, the results showing that laparoscopy is associated with a more static posture of the neck and torso, but at the same time it provokes more incorrect movements in the upper extremity. Maintaining these static postures for prolonged periods of time is the cause of greater muscle fatigue

The design of laparoscopic surgery instruments, a specific type of tools with which the surgeon works during a minimally invasive surgical intervention of the pelvic-abdominal cavity is a fundamental aspect for performing his work since these elements have a considerable ergonomic impact on many of the tasks performed by the surgeon. There are various ergonomic studies on the design of tools on a general level in which fundamentally the following objectives are considered:
- Adapting them to the task, considering the grip requirements (of force or precision) in the operations.
- Adapting them to the workers, taking into account hand anthropometry, the capacity of force and mobility and other characteristics of people when choosing shapes, sizes, materials and operating mode of the instrument.

Today, there is a wide variety in terms of the types of laparoscopic instruments. Four essential groups are identified according to the function thereof:
- Grasping and intense tissue manipulation.
- Electrosurgery and coagulation.
- Suction and irrigation.
- Suture.

According to the type of handle, there are, among others:
- Axial handle
- Shank handle
- Multifunctional handle
- Ring Handle

However, the instruments differ not only in the function that they carry out but in the handle, length and design of the tip. Laparoscopic techniques and procedures, as well as the varieties of instruments have evolved in recent years, the appearance and operation thereof however continue to be similar to the initial designs.

Earlier works show that the configuration of the gripping systems of laparoscopic instruments has an inadequate design which usually translates into the onset of muscle fatigue, discomfort and even paresthesia. The use of more ergonomic handles contributes to an increase in the speed and quality when executing laparoscopic exercises.

Adapting the surgical instruments to the type of operation and to the capacities and characteristics of the surgeons has the following benefits:
- Reducing overloading the joints, ligaments and muscles of the upper limbs, avoiding forced postures and repetitive movements, minimizing the force necessary for using the instrument.
- Improving performance and efficacy of the operation performed with the instrument, as well as improving the quality of the associated operation. Ergonomic-type improvements in the design of the working systems are economically feasible in terms of improving the performance and reducing operating costs.

A common problem in the use of laparoscopic instruments is the size of the handle. Since surgical interventions are performed by surgeons of both sexes with different physical complexions and therefore with different hand sizes, the instruments require being able to be adapted to this characteristic of each professional. The size of the surgical glove helps to know the relationship between hand size and the grip of the handles. Larger sizes are common in men while smaller sizes are common in women. As a consequence, the women participating in the studies have more difficulties using laparoscopic instruments. Inventions with application numbers PCT W02006/071120 and W02006/071121 intend to solve this problem, which inventions consist of instruments for use in laparoscopic surgery, each including at least one adjustable portion in the rear part of the handles by means of the corresponding mechanisms for the purpose of adapting it to different hand sizes. Both instruments place the orientation wheel of the distal tool at the distal end of the handle for its actuation by means of the index finger.

PCT application number W02006/071121 discloses a mechanism formed by three linkages which transform the partial rotational movement of the trigger into a linear movement of the pin of the working shaft which opens and closes the distal tool. The latter remains open in the resting state due to the action of a spring.

With this solution, the necessary displacement distances require the intermediate linkage to start from the lower area of the trigger and the overall thickness of the pull solution is large.

The present invention establishes an alternative pull mode, with minimal friction parts resulting in a very precise movement control and a much higher degree of compaction.

### Description of the Invention

The present invention is an apparatus for laparoscopic surgery which allows acting on an actuation tool. This actuation tool has rotating capacity and at least two degrees of opening. It is typical for it to incorporate a notched opening that can be opened up to a determined point and further maintain control over its angular position.

For allowing moving and controlling these two degrees of freedom, the apparatus of the present invention comprises:
- *a main body which in turn has a gripping handle of the apparatus*,
- *an actuation tool which allows at least two degrees of opening and is orientable by means of rotation*,
- *a tubular body for fastening the main body with the actuation tool*,
   Up to this point, the more visible elements of the apparatus together with the control trigger for closing the tool and the control which allows controlling the angular position thereof are presented.
- *a pin arranged inside the tubular body with rotating and axial displacement capacity with respect to the tubular body where one end of the pin is linked to the actuation tool such that the axial displacement of the pin determines the degree of opening of the actuation tool and the rotation of the pin determines the orientation by means of rotating the same actuation tool*,
   This control mode is the one used in the present invention. A control for opening the tool based on the axial displacement of the pin with respect to the tubular body; and, a control of the rotation of the element linked angularly to the tool are used in the prior art. In the mentioned closest prior art, the rotation is established by the outer tubular body whereas in the present invention, the pin is responsible for the rotation. This form of acting on the tool conditions the structure and kinematics of the elements which act on the pin.
- *a trigger pivotally linked with respect to the main body and joined to the pin such that the rotation of the trigger pulls the pin*,
   The trigger is one of the actuation means for acting on the tool. It is the actuation means which allows controlling the degree of opening of the tool. Its actuation is carried out by executing a backwards force to pull the pin.
- *a control with rotating capacity and linked angularly to the pin such that the rotation of the control is transmitted to the pin*;
   The control is the second actuation means which acts on the second degree of freedom of the tool. In the present invention, the actuation on the control is transmitted as an angular stress on the pin which in turn rotates and controls the orientation of the tool.

***wherein** it additionally comprises an axial transmission part which in turn comprises a ball joint and a bar element, where the bar element is fixed angularly to the control with rotating capacity and the ball joint is fixed both angularly and axially to the end of the pin opposite the actuation tool; and, wherein the trigger comprises a head with a housing for the ball joint such that the pull of the trigger on the pin is exerted through the ball joint*.

These last two features are what determine the mode of simultaneous actuation of the two degrees of freedom, axial displacement and rotation, on the same element, the pin. A part referred to as axial transmission part which in turn comprises the following parts: a ball joint and a bar, is used.

The axial transmission part is joined to the pin and acts on it, transmitting thereto two types of forces, torsional force to cause rotation and pull force to cause axial displacement.

The trigger pulls the pin, exerting the force on the ball joint. The attachment with the latter is such that it does not prevent the rotation imparted by the control.

The control in turn acts on the bar such that the link between the bar and the control is such that the relative rotation therebetween is limited.

The particular modes for carrying out these linkages according to dependent claims 2 to 9 are incorporated by reference to this description. Embodiments in which solutions of different dependent claims, with configurations applicable to the same invention, are incorporated in a combined form are also considered incorporated by reference.

The details shown in an embodiment are described in the detailed description of the invention.

### Description of the Drawings

The foregoing and other advantages and features will be better understood from the following detailed description according to a preferred embodiment, given only by way of illustrative and non-limiting example with reference to the attached drawings.
Figure 1 shows an embodiment of the apparatus according to one perspective.
Figure 2 shows the same embodiment with the handle being sectioned to allow observing the inside with the inner parts and with an arrangement of the elements according to a resting position of the trigger.
Figure 3 shows the same embodiment with the handle being sectioned to allow observing the inside with the inner parts and with an arrangement of the elements according to a displaced position of the trigger upon having pulled it.
Figure 4 shows a perspective view of the same embodiment also with the handle being sectioned to allow observing the inside with the inner parts and details of how they are linked to one another.

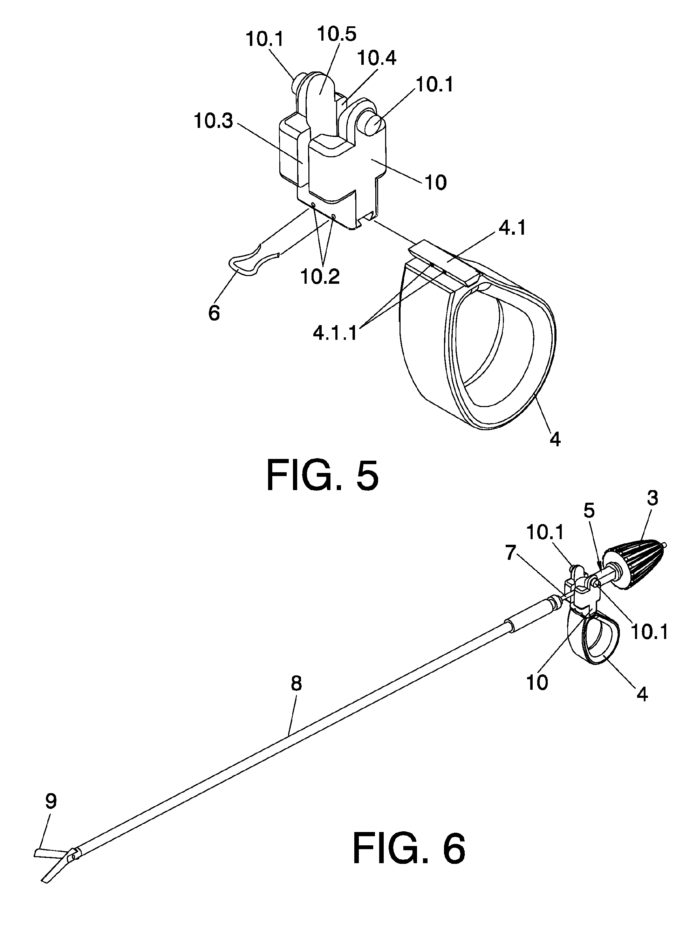

Figure 5 shows a detail of the head of the trigger and the trigger, also of the particular mode of mutual linkage used.
Figure 6 shows a perspective view of the inner parts as well as the tool after having removed the parts of the handle which cover said inner parts. The view is a perspective view.
Figure 7 shows a perspective view of the pin as well as the axial transmission part and the control. The parts have been arranged according to an exploded perspective to better observe the configurative details.

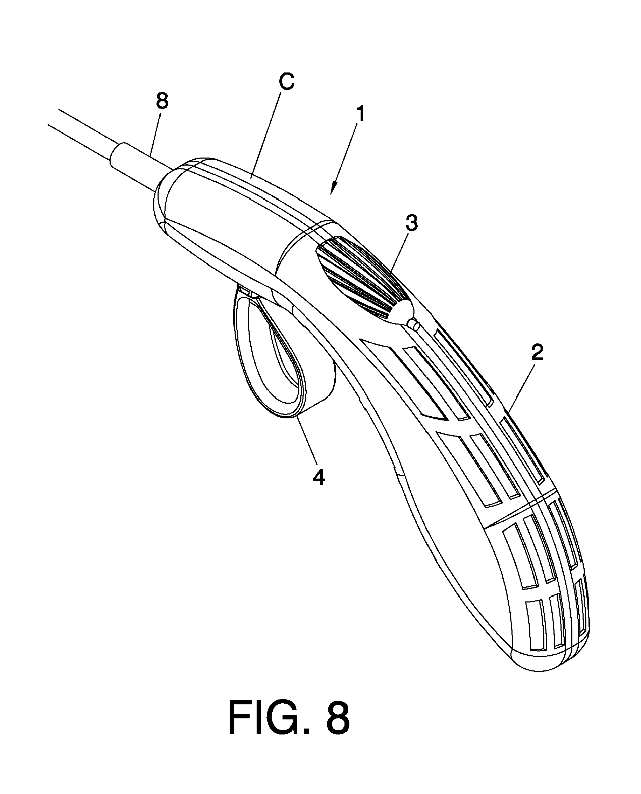

Figure 8 shows the same embodiment being assembled to observe the ergonomic configuration which allows the technical solution used.

### Detailed Description of the Invention

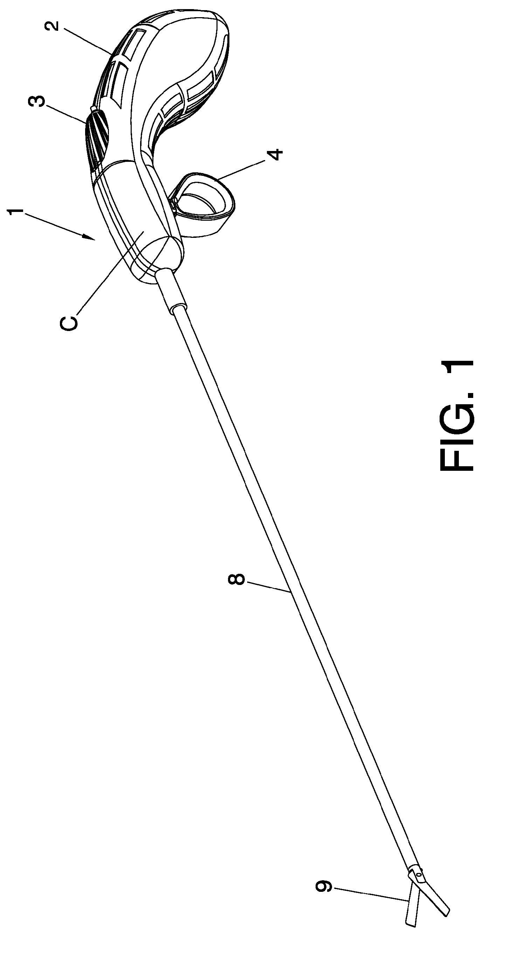

Figure 1 shows an embodiment of the device (1) according to a perspective view which allows observing the outer elements. The device (1) has an actuation tool (9) at one end which serves to carry out the different actions inside the body to be operated on by means of laparoscopy. This actuation tool (9) according to this embodiment is a clamp with two degrees of freedom: rotation and opening and closing.

The actuation tool (9) is located at the end of a tubular body (8) such that it has rotating capacity with respect to this tubular body (8). The base of the actuation tool (9) is integral with a pin (7) arranged inside the tubular body (8) and the pin is not seen in this figure.

As will be described below, the rotation of the actuation tool (9) is achieved by means of the rotational movement of the pin (7); and, the opening or closing of the clamp is produced by the axial displacement of the pin (7) with respect to the tubular body (8) in which it is housed.

The other end of the tubular body (8) is integral with the main body (C) of the device (1) where the main components allowing the actuation and control of the actuation tool (9) are located.

The trigger (4) which opens and closes the actuation tool (9) which is actuated by means of the index finger of the user is located in the inner part of the main body (C).

The same main body (C) is continuously prolonged in its rear part, becoming thicker until configuring the handle (2). A mainly conical and slightly dished sector of surface corresponding to the control (3) is observed in the transition between the main body (C) and the handle (2). This configuration and position of the control (3) allows exerting force with the thumb to either side, achieving its rotation and therefore also the rotation of the actuation tool (9). Since the surface in this transition between the main body (C) and the handle (2) has a curvature coinciding to a great extent with the curvature of the surface of the control (3) the degree of integration between both bodies is very high and allows very comfortable and effective handling.

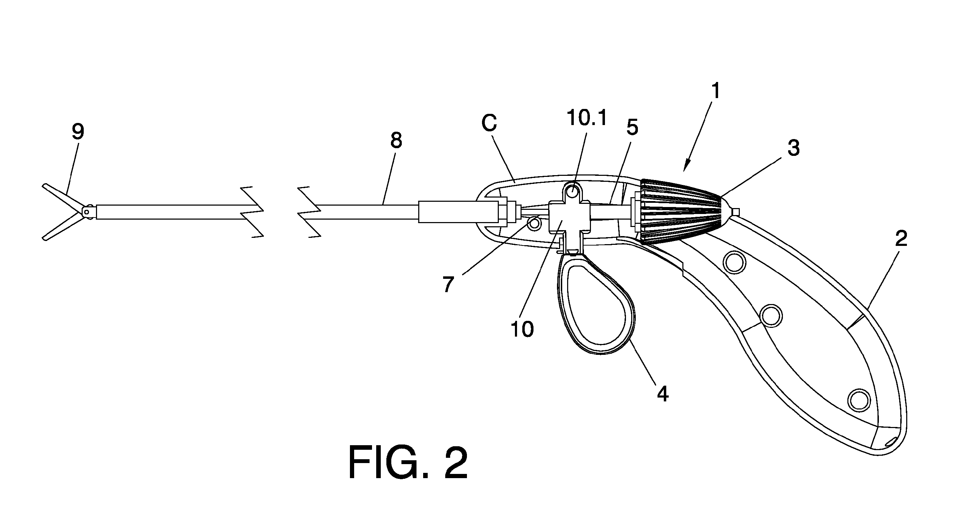

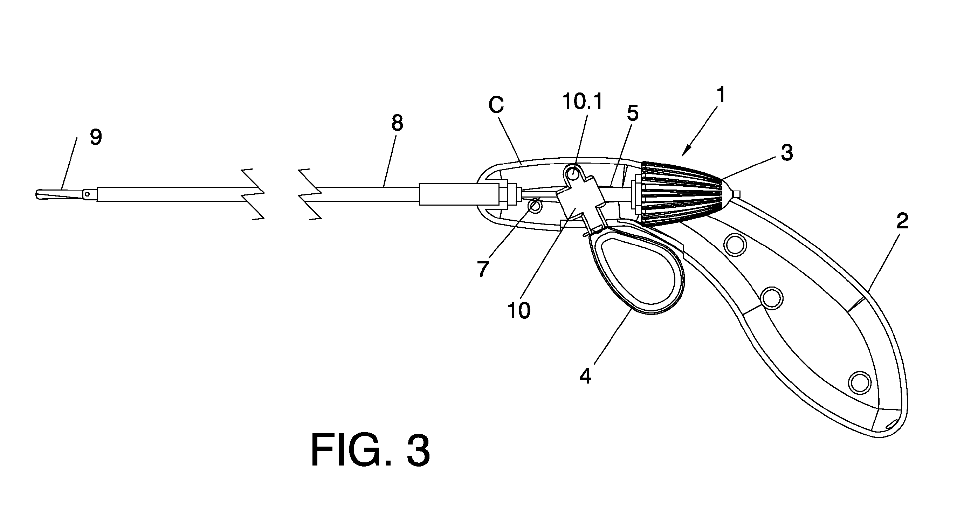

Figures 2 and 3 show a section of the main body (C) and the handle (2) to allow observing the parts contained therein, being shown with the trigger (4) in the resting position (in Figure 2) and with the trigger (4) actuated (in Figure 3).

Both figures show how the pin (7) which is housed in the tubular body (8) is prolonged until reaching the inside of the main body (C). The pin (7) is aligned with the axis of rotation of the control (3), except for small deviations due to the operating mode. The rotation of the control (3) will cause the rotation of the pin (7) which in turn causes the rotation of the actuation tool (9).

This movement is independent of the axial movement of the pin (7) which is responsible for opening and closing the actuation tool (9).

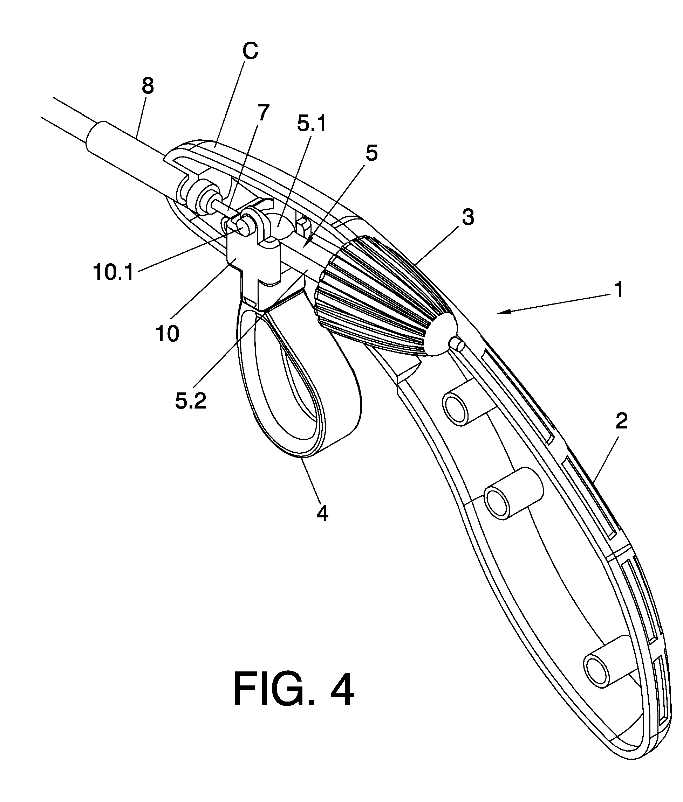

According to Figure 4, where the same section is observed in perspective, it is possible to identify in this embodiment how the trigger (4) and the head (10) are two independent parts linked by a dovetail joint (4.1). This joint is secured by means of a clamp (6) entering holes (10.2) of the head (10) which communicate with other holes (4.1.1) in the dovetail (4.1) of the trigger (4). The head (10) has in the upper part two pivots (10.1) which, housed in respective slots of the main body (C), allow the rotation of the trigger (4) and the head (10), which move as a single body.

The fact that the trigger (4) is interchangeable increases the range of possibilities of working triggers (4) which can be used with the same device not only for the index finger, but also for being able to work with one or more fingers positioned on said trigger (4). These interchangeable triggers (4), for example ring-shaped triggers, can be removed by the surgeon from the main body (C) at will according to his preferences.

The head (10) is located below (the lower position is considered as below according to the orientation shown in the figure) the axis of rotation which the pivots (10.1) define. In this manner, upon pulling the trigger (4) towards the handle (2) by applying force with the index finger, the head (10) is also displaced backwards.

The inside of the head (10) is partially hollow with two cylindrical surfaces (10.5) which allow housing a spherical body.

This spherical body can be vertically displaced (also according to the position shown in this figure) sliding along the two cylindrical surfaces (10.5).

A first groove (10.3) for the passage of the pin (7) is shown in the front part of the head (10).There is a second groove (10.4) for the passage of the part referred to as axial transmission part (5) in the rear part, in opposition to this first groove (10.3).

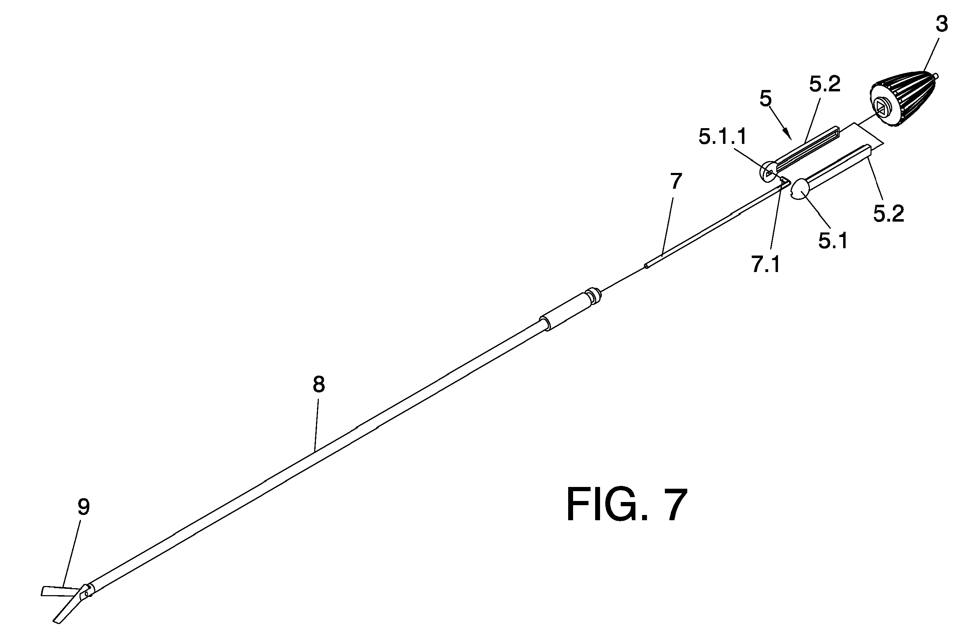

The axial transmission part (5) can be observed in detail in Figure 7 where a ball joint (5.1) and a bar element (5.2) integral with one another are identified. The ball joint (5.1) is the spherical body which enters the housing of the head (10) formed between the cylindrical surfaces (10.5).

Figure 7 also allows observing how the axial transmission part (5) in this embodiment is in turn made up of and formed by two halves trapping the end (7.1) of the pin (7). The end (7.1) of the pin (7) is curved and enters an internal housing (5.1.1) of one of the halves of the axial transmission part (5). This attachment makes two parts, the pin (7) and the axial transmission part (5), integral with one another. The rotation of one part causes the rotation of the other and the axial displacement of one part causes the axial displacement of the other.

The bar element (5.2) has an outer polyhedral shape or any other shape which prevents the relative rotation with the control (3) which houses the end of the bar element (5.2). In this embodiment, the section is a square section. Upon entering the essentially conical body which gives rise to the control (3), the rotation also imparts the rotation of the other as well, but mutual displacement is not prevented.

Also according to Figure 4, it can be observed how the axial transmission part (5) is linked to the head (10) such that the displacement of the trigger (4) also causes the displacement of the ball joint (5.1). Although the movement of the trigger (4) is rotational movement about the pivots (10.1) in the head (10), the possibility of relative displacement of the ball joint (5.1) along the cylindrical surfaces (10.5) allows the ball joint (5.1) to only describe an axial trajectory exerting a pull force on the pin (7) without the ball joint necessarily following the arched trajectory.

In turn, the seat in the form of a cylindrical surface (10.5) and the spherical shape of the ball joint (5.1) allow transmitting the rotation imparted by the control (3) on the bar element (5.2) to the pin (7) completely independent of the axial position.

## Claims

1. An apparatus for laparoscopic surgery comprising:
• a main body (C) which in turn has a gripping handle (2) of the apparatus,
• an actuation tool (9) which allows at least two degrees of opening and is orientable by means of rotation,
• a tubular body (8) for fastening the main body (C) with the actuation tool (9),
• a pin (7) arranged inside the tubular body (8) with rotating and axial displacement capacity with respect to the tubular body (8) where one end of the pin (7) is linked to the actuation tool (9) such that the axial displacement of the pin (7) determines the degree of opening of the actuation tool (9) and the rotation of the pin (7) determines the orientation by means of rotating the same actuation tool (9),
• a trigger (4) pivotally linked with respect to the main body (C) and joined to the pin (7) such that the rotation of the trigger (4) pulls the pin (7),
• a control (3) with rotating capacity and linked angularly to the pin (7) such that the rotation of the control (3) is transmitted to the pin (7);
**characterized in that** it additionally comprises an axial transmission part (5) which in turn comprises a ball joint (5.1) and a bar element (5.2) where the bar element (5.2) is angularly fixed to the control (3) with rotating capacity and the ball joint (5.1) is fixed both angularly and axially to the end of the pin (7) opposite to the actuation tool (9); and,
where the trigger (4) comprises a head (10) with a housing for the ball joint (5.1) such that the pull of the trigger (4) on the pin (7) is exerted through the ball joint (5.1).

2. The apparatus according to claim 1, **characterized in that** the housing of the head (10) for the ball joint (5.1) is formed by two sectors of cylindrical surface such that it allows the displacement of the ball joint (5.1) in a direction essentially perpendicular to the axis of the bar (5.2).

3. The apparatus according to claim 1, **characterized in that**:
• the control (3) is essentially conical-shaped,
• the handle (2) is configured in continuity with the main body (C) such that the part of the conical surface of the control (3) is also in continuity with the surface of the handle (2) and the main body (C).

4. The apparatus according to claim 1, **characterized in that** the trigger (4) and the head (10) are two independent parts linked by a dovetail joint.

5. The apparatus according to claim 4, **characterized in that** the dovetail joint is secured by means of a clamp (6) the ends of which traverse perforations (4.1.1, 10.2) both of the head (10) and of the trigger (4).

6. The apparatus according to claim 1, **characterized in that** the axial transmission part (5) is formed by two half portions.

7. The apparatus according to claim 6, **characterized in that** the separation of the two half parts is according to a plane passing through the axis of the bar (5.2).

8. The apparatus according to claim 6 or 7, **characterized in that** on the surface or the surfaces of separation between both parts, there is a housing (5.1.1) where part of the end (7.1) of the pin (7) is housed for the anchoring thereof.

9. The apparatus according to claim 1, **characterized in that** the angular fixation between the control (3) and the bar (5.2) allows the relative axial displacement therebetween.
